# EUROPEAN PATENT APPLICATION

(11) **EP 4 843 506 A1**
(43) Date of publication of application: **25.11.2026**
(21) Application number: 25177591.2
(22) Date of filing: 20.05.2025
(51) Int. Cl.: A61B 6/04, A61B 6/06, A61B 6/46, A61B 6/50, A61B 5/00

(54) **RADIOGRAPHY APPARATUS**

(71) Applicant: Koninklijke Philips N.V., 5656 AE Eindhoven (NL)
(72) Inventor: MENSER, Bernd, Eindhoven (NL); KROENKE-HILLE, Sven, Eindhoven (NL); HAWELLEK, Benjamin, 5656AG Eindhoven (NL); BYSTROV, Daniel, Eindhoven (NL); VON BERG, Jens, Eindhoven (NL); MAY, Jan Marek, Eindhoven (NL); WIEBERNEIT, Nataly, Eindhoven (NL); ZLOTOPOLSKI, Melanie, Eindhoven (NL)
(74) Representative: Philips Intellectual Property & Standards

(57) **Abstract**

The present disclosure describes a radiography apparatus comprising a controller configured to identify a landmark feature from a first radiographic image; and to determine a spatial correspondence of a first radiographic image sensor with respect to a second positional image sensor. The controller is further configured to, prior to a second data acquisition, control a display (128) to display a user interface (130) comprising displaying at least one of: a live image (132) of a target currently captured by the second image sensor overlaid with the identified landmark feature (134) at a location based on the determined spatial correspondence; and a first positional image overlaid with the identified landmark feature at a location based on the determined spatial correspondence.

## Description

### FIELD OF THE INVENTION

The subject-matter of the present disclosure relates to radiography, and in particular obtaining accurate images from a radiography apparatus, a radiography apparatus therefor, and a method for controlling the radiography apparatus.

### BACKGROUND OF THE INVENTION

Radiographic imaging, e.g., X-ray imaging, requires proper patient positioning relative to the radiography system for acquiring images of diagnostic quality. For musculoskeletal exams, achieving an appropriate patient pose often requires several image acquisitions until an image sufficient quality is achieved. Many radiographic imaging procedures are follow-up examinations to monitor the progression of a disease or therapy, e.g., to monitor orthopedic implants. In such monitoring, it is desirable to maintain consistency in radiographic images taken at different stages in the monitoring of the progression of the disease or therapy. More consistent radiographic images enable improved monitoring of the progression of the disease or therapy.

It is therefore an aim of the subject-matter of the present disclosure to improve on the prior art.

### SUMMARY OF THE INVENTION

According to a first aspect of the present invention, there is provided a radiography apparatus. The apparatus comprises a radiographic imager configured to capture a radiographic image of a target, the radiographic imager comprising a radiation generator and a first image sensor. The apparatus comprises a positional imager comprising a second image sensor configured to capture a positional image of the target (such as a visible light image). The apparatus comprises a display and a memory. The apparatus comprises a controller configured to obtain a first radiographic image of the target from the memory, wherein the first radiographic image was captured in a first data acquisition, and the first radiographic image comprises a landmark feature. The controller is further configured to identify the landmark feature in the first radiographic image. The controller is further configured to determine a spatial correspondence of the first image sensor with respect to the second image sensor. The controller is further configured to, prior to a second data acquisition, control the display to display a user interface comprising displaying a live image of the target currently captured by the second image sensor overlaid with the identified landmark feature at a location based on the determined spatial correspondence. In this way, the identified landmark feature is displayed at a location corresponding to an internal location of the landmark feature.

In an example of the first aspect, the positional imager comprises a depth sensor.

In an example of the first aspect, the landmark feature is identified by inputting the first radiographic image into a trained machine learning model.

In an example of the first aspect, the identified landmark feature is an implant, for example an orthopaedic implant.

In an example of the first aspect, the first image sensor comprises one or more markers, and determining the spatial correspondence of the first image sensor comprises determining the location of the markers within the live image.

In an example of the first aspect, the determining the spatial correspondence of the first image sensor comprises analysing an anatomy of the target in the live image and comparing to an anatomy in the first radiographic image.

In an example of the first aspect, the controller is further configured to obtain a first positional image corresponding to the first radiographic image from the memory, wherein the first positional image was captured in the first data acquisition; and to control the display to display the first positional image. Optionally, the first positional image is registered to the first radiographic image, and the first radiographic image can also be displayed with the first positional image, for example, overlaid onto the first positional image.

In an example of the first aspect, the displaying the live image overlaid with the identified landmark feature comprises displaying a bounding box corresponding to the landmark feature.

In an example of the first aspect, the displaying the live image overlaid with the identified landmark feature comprises displaying a generated representation the landmark feature.

In an example of the first aspect, the radiographic imager further comprises a collimator, and the controller is further configured to adjust a collimation parameter. For example, the collimation parameter may be adjusted based on the displayed user interface or based on the identified landmark feature, e.g., based on the location of the identified landmark feature.

In an example of the first aspect, the controller is further configured to: determine a pose of the target captured in the first radiographic image; determine a pose of the target captured in the live image; determine a pose correction based on the determined pose in the first radiographic image and the determined pose in the live image, and the user interface comprises displaying information corresponding to the determined pose correction.

In an example of the first aspect, the radiation generator is provided on a moveable head part of the apparatus, and the second image sensor is also provided on the moveable head.

In an example of the first aspect, the display is provided on the moveable head.

According to a second aspect of the present invention, there is provided a radiography apparatus. The apparatus comprises a radiographic imager configured to capture a radiographic image of a target, the radiographic imager comprising a radiation generator and a first image sensor. The apparatus comprises a positional imager comprising a second image sensor configured to capture a positional image of the target (such as a visible light image, e.g., a camera). The apparatus comprises a display and a memory. The apparatus comprises a controller configured to obtain a first radiographic image of the target from the memory, wherein the first radiographic image was captured in a first data acquisition, and the first radiographic image comprises a landmark feature. The controller is further configured to obtain a first positional image corresponding to the first radiographic image from the memory, wherein the first positional image was captured in the first data acquisition. The controller is further configured to identify the landmark feature in the first radiographic image. The controller is further configured to determine a spatial correspondence of the first image sensor with respect to the second image sensor. The controller is further configured to, prior to a second data acquisition, control the display to display a user interface comprising displaying the first positional image overlaid with the identified landmark feature at a location based on the determined spatial correspondence. In this way, the identified landmark feature is displayed at a location corresponding to an internal location of the landmark feature.

In an example of the second aspect, the positional imager comprises a depth sensor.

In an example of the second aspect, the landmark feature is identified by inputting the first radiographic image into a trained machine learning model.

In an example, the identified landmark feature is an implant, for example an orthopaedic implant.

In an example of the second aspect, the first image sensor comprises one or more markers, and determining the spatial correspondence of the first image sensor comprises determining the location of the markers within the first positional image.

In an example of the second aspect, the determining the spatial correspondence of the first image sensor comprises analysing an anatomy of the target in the first positional image and comparing to an anatomy in the first radiographic image.

In an example of the second aspect, the controller is further configured to control the display to display a live image of the target currently captured by the second image sensor.

In an example of the second aspect, the displaying the first positional image overlaid with the identified landmark feature comprises displaying a bounding box corresponding to the landmark feature.

In an example of the second aspect, the displaying the first positional image overlaid with the identified landmark feature comprises displaying a generated representation the landmark feature.

In an example of the second aspect, the radiographic imager further comprises a collimator, and the controller is further configured to adjust a collimation parameter. For example, the collimation parameter may be adjusted based on the displayed user interface or based on the identified landmark feature, e.g., based on the location of the identified landmark feature.

In an example of the second aspect, the controller is further configured to: determine a pose of the target captured in the first radiographic image; determine a pose of the target captured in the first positional image; determine a pose correction based on the determined pose in the first radiographic image and the determined pose in the first positional image, and the user interface comprises displaying information corresponding to the determined pose correction.

In an example of the second aspect, the radiation generator is provided on a moveable head part of the apparatus, and the second image sensor is also provided on the moveable head.

In an example of the second aspect, the display is provided on the moveable head.

According to a third aspect of the present invention, there is provided a radiography apparatus. The apparatus comprises a radiographic imager configured to capture a radiographic image of a target, the radiographic imager comprising a radiation generator and a first image sensor. The apparatus comprises a positional imager comprising a second image sensor configured to capture a positional image of the target (such as a visible light image, e.g., a camera). The apparatus comprises a display and a memory. The apparatus comprises a controller configured to obtain a first radiographic image of the target from the memory, wherein the first radiographic image was captured in a first data acquisition, and the first radiographic image comprises a landmark feature. The controller is further configured to obtain a first positional image corresponding to the first radiographic image from the memory, wherein the first positional image was captured in the first data acquisition. The controller is further configured to identify the landmark feature in the first radiographic image. The controller is further configured to determine a spatial correspondence of the first image sensor with respect to the second image sensor. The controller is further configured to, prior to a second data acquisition, control the display to display a user interface comprising displaying at least one of: a live image of the target currently captured by the second image sensor overlaid with the identified landmark feature at a location based on the determined spatial correspondence; and the first positional image overlaid with the identified landmark feature at a location based on the determined spatial correspondence. In this way, the identified landmark feature is displayed at a location corresponding to an internal location of the landmark feature.

In an example of the third aspect, the positional imager comprises a depth sensor.

In an example of the third aspect, the landmark feature is identified by inputting the first radiographic image into a trained machine learning model.

In an example of the third aspect, the identified landmark feature is an implant, for example an orthopaedic implant.

In an example of the third aspect, the first image sensor comprises one or more markers, and determining the spatial correspondence of the first image sensor comprises determining the location of the markers within the live image or the first positional image.

In an example of the third aspect, the determining the spatial correspondence of the first image sensor comprises analysing an anatomy of the target in the live image or the first positional image and comparing to an anatomy in the first radiographic image.

In an example of the third aspect, when the live image of the target currently captured by the second image sensor overlaid with the identified landmark feature at the location based on the determined spatial correspondence is displayed, the controller is further configured to control the display to display the first positional image.

In an example of the third aspect, when the first positional image overlaid with the identified landmark feature at the location based on the determined spatial correspondence is displayed, the controller is further configured to control the display to display the live image of the target currently captured by the second image sensor.

In an example of the third aspect, the displaying the live image or the first positional image overlaid with the identified landmark feature comprises displaying a bounding box corresponding to the landmark feature.

In an example of the third aspect, the displaying the live image or the first positional image overlaid with the identified landmark feature comprises displaying a generated representation the landmark feature.

In an example of the third aspect, the radiographic imager further comprises a collimator, and the controller is further configured to adjust a collimation parameter. For example, the collimation parameter may be adjusted based on the displayed user interface or based on the identified landmark feature, e.g., based on the location of the identified landmark feature.

In an example of the third aspect, the controller is further configured to: determine a pose of the target captured in the first radiographic image; determine a pose of the target captured in at least one of the live image and the first positional image; determine a pose correction based on the determined pose in the first radiographic image and the determined pose in the at least one of the live image and the first positional image, and the user interface comprises displaying information corresponding to the determined pose correction.

In an example of the third aspect, the radiation generator is provided on a moveable head part of the apparatus, and the second image sensor is also provided on the moveable head.

In an example of the third aspect, the display is provided on the moveable head.

In related aspects of the invention there is provided a method for controlling a radiography apparatus. The method comprises obtaining a first radiographic image of a target from a memory, wherein the first radiographic image was captured by a first image sensor in a first data acquisition, and the first radiographic image comprises a landmark feature. The method comprises identifying the landmark feature in the first radiographic image. The method comprises determining a spatial correspondence of the first image sensor with respect to a second image sensor configured to capture a positional image of the target. The method comprises, prior to a second data acquisition, controlling the display to display a user interface comprising displaying at least one of: a live image of the target currently captured by the second image sensor and overlaid with the identified landmark feature at a location based on the determined spatial correspondence; and a first positional image overlaid with the identified landmark feature at a location based on the determined spatial correspondence. In this way, the identified landmark feature is displayed at a location corresponding to an internal location of the landmark feature. In aspects in which the method comprises displaying the first positional image overlaid with the identified landmark feature, the method further comprises obtaining the first positional image corresponding to the first radiographic image from the memory, wherein the first positional image was captured in the first data acquisition.

In an example, the method further comprises adjusting a collimation parameter of a collimator. For example, the collimation parameter may be adjusted based on the displayed user interface or based on the identified landmark feature, e.g., based on the location of the identified landmark feature.

It will be appreciated that the invention also extends to method aspects corresponding to the apparatus aspects and examples outlined above.
These and other aspects of the present invention will be apparent from and elucidated with reference to the embodiments described hereinafter.

### BRIEF DESCRIPTION OF DRAWINGS

The embodiments of the present inventions may be best understood with reference to the accompanying figures, in which:
Fig. 1 shows a schematic of a radiography apparatus;
Fig. 2 shows an example display and user interface;
Fig. 3 shows another example display and user interface;
Fig. 4 shows another example display and user interface;
Fig. 5 shows another example display and user interface; and
Fig. 6 shows an example image transformation.

### DETAILED DESCRIPTION OF EMBODIMENTS

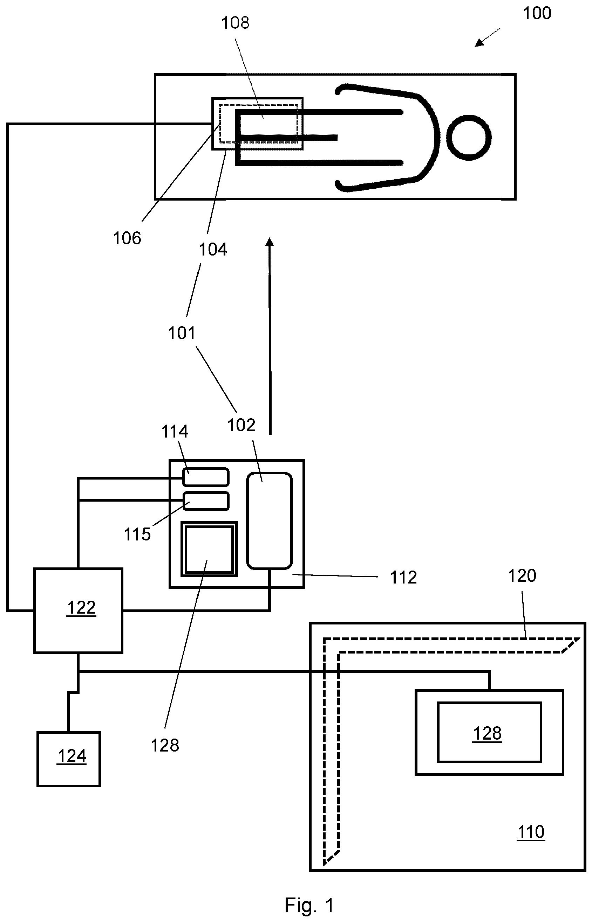

With reference to Fig. 1, there is shown an example radiography apparatus (or system) 100. The radiography apparatus 100 is based around the emission of radiation and the subsequent imaging of that radiation. For example, the radiography apparatus 100 may be embodied as an X-ray imaging system, and other variants of imaging systems using ionizing radiation.

Suitably, the apparatus 100 comprises a radiographic imager 101; for example, comprising a radiation generator 102 and a radiographic image sensor 104, also termed a first image sensor 104 herein. For example, in the X-ray imaging system, the radiation generator 102 is configured to generate X-rays and the first image sensor 104 is correspondingly configured to capture an X-ray image. If the generator 102 is configured to generate a different form of radiation, the first image sensor 104 is configured accordingly. As will be familiar to those in the art, the arrangement of the radiographic imager 101 defines a target area 106 in which a target 108, such as a patient, or part thereof, can be positioned in order to capture a radiographic image based on the penetration of radiation through the target 108 in the target area 106. Put another way, the first image sensor 104 is configured to capture a radiographic image of the target area 106. In some examples, the first image sensor 104 is provided as a separate unit configured to communicate, preferably wirelessly, with other components of the apparatus 100.

In some examples, radiation generator 102 and first image sensor 104 are provided in a fixed arrangement, with the target 108 being moved with respect to the generator 102 and sensor 104 to allow for imaging of different parts of the target 108. Accordingly, in these examples, the target area 106 to be imaged is fixed in position.

In other examples, the radiation generator 102 and first image sensor 104 are coupled to suitable movement means such that one or both of the first image sensor 104 and radiation generator 102 may be moved relative to the target 108. That is, in these examples, the target 108 is substantially fixed in position, while the target area 106 to be imaged may be re-positioned according to the moveable arrangement between the first image sensor 104 and radiation generator 102.

In one example of a moveable radiation generator 102 and first image sensor 104, the first image sensor 104 is coupled to one or more rails to provide lateral motion, while the generator 102 is coupled to a moveable arm.

In another example of a moveable radiation generator 102 and first image sensor 104, the radiation generator 102 is provided on a suitably moveable head part, or tube head, 112. Meanwhile, the first image sensor 104 is provided as a separate unit configured to communicate wirelessly with other components of the apparatus 100. To capture an image of the target 108, the tube head 112 is aligned with the target 108 and the first image sensor 104 is freely positioned behind the target 108 to capture an image of the radiation.

In the example shown in Fig. 1, the apparatus 100 comprises a control station 110, from which control parameters and functions of the radiographic imager 101 may be set. The control station 110 is disposed separated from the target area 106 of the radiographic imager 101 so as to limit the potential for exposure of the radiographer of the apparatus 100 to radiation. In some examples, the control station 110 is shielded from the imager 101 (more specifically, the radiation generator 102 and target area 106 therefor) by a radiation shield 120.

In some examples, the apparatus 100 comprises a display 128 for viewing images captured by the radiographic imager 101. As shown in Fig. 1, in some examples the display 128 may be provided at the control station 110; in other examples the display 128 may be provided on the tube head 112.

The apparatus 100 comprises a positional imager comprising a second image sensor 114 having a field of view which encompasses the target area 106. That is, the second image sensor 114 can capture positional images of the target 108 within the target area 106. The radiographer can use the second image sensor 114 to instruct adjustment of the target 108 and/or apparatus 100 in order to allow for correct imaging of the target 108.

The second image sensor 114 may be configured to capture light at any suitable wavelength that allows for positioning the target 108. In one example, the second image sensor 114 is configured to capture visible light; suitably, in such examples, the second image sensor 114 may be embodied as an RGB camera. In another example, the second image sensor 114 is configured to capture infra-red light, and may therefore be embodied as an infra-red camera. In yet another example, the second image sensor 114 is configured to capture depth information; suitably, in such examples the second image sensor 114 may be part of an RGB depth (RGB-D) camera.

In some examples, the second image sensor 114 is integrated as part of the radiographic imager 101. That is, as a component of the respective part of the imager 101, such as the radiation generator 102, or a related component thereof. In the present example, the second image sensor 114 is integrated as part of the tube head 112 of the apparatus 100, such that its position substantially corresponds to the position of the radiation generator 102, and its field of view substantially corresponds with the direction of radiation emission from the generator 102, allowing the second image sensor 114 to capture an image of the target area 106.

Suitably, the apparatus 100 comprises a collimator to change the size of a beam of radiation emitted by the radiation generator 102. The collimator may be a motorised automatic collimator or a manual collimator. The collimator limits radiation exposure to the target area 106, i.e., reducing the amount of radiation to which the patient or the operator of the apparatus (i.e., the radiographer) is exposed, and reducing the amount of scatter radiation. The collimator includes collimator shutters which are used to control the beam of radiation, e.g., to change the size of the beam of radiation. In the case of a motorised automatic collimator, the collimator shutters can be moved automatically into a position. The position and orientation of the collimator shutters can be measured and stored as collimation parameters.

In some examples, the apparatus 100 comprises a light 115. The light 115 is configured to emit a visible light beam to indicate a position and orientation of the target area 106. When the apparatus 100 is in a configuration for capturing an image, the light beam is emitted towards the first image sensor 104, such that the first image sensor 104 has an area illuminated by the light 115. In the present example, the light 115 is integrated as part of the tube head 112 of the apparatus 100, such that its position substantially corresponds to the position of the radiation generator 102 and the second image sensor 114, and the light beam substantially corresponds with the direction of radiation emission from the generator 102, and the field of view of the second image sensor 114. In this way, the illuminated area can be used to ensure that the apparatus 100 is in a suitable configuration for capturing a radiographic image. In some examples, the light is included in the collimator, and the light beam and radiation beam are emitted from the same or substantially corresponding apertures, so that the light beam corresponds to the radiation beam.

The apparatus 100 comprises a controller 122, e.g., a processor, configured to control an operating state of the apparatus 100. One example operating state is an image capturing state, i.e., a data acquisition state, in which the device is able to capture an image. That is, the controller 122 is configured to control a data acquisition comprising controlling the radiographic imager 101 to capture a radiographic image of the target area 106. Put another way, the controller 122 controls the radiation generator 102 to emit radiation and the first image sensor 104 to record an image of an irradiated area of the first image sensor 104. The controller 122 may also control the second image sensor 114 to also capture an image including the target area 106. The captured second image sensor 114 data may be an image, a video, or the like. Furthermore, at substantially the same time, the controller 122 may control the light 115 to emit the light beam, such that an area illuminated by the light beam is visible in the captured second image sensor data.

Suitably, the acquired data is stored in a memory 124. In some examples, the memory 124 is local to the apparatus 100, e.g., an internal memory storage. In other examples, the memory 124 is external to the apparatus 100. In the case of an external memory, the apparatus 100 may comprise suitable transceiver circuitry for communication with the external memory 124.

It will be appreciated that, to improve monitoring of the progression of a disease or therapy, it is desirable to maintain consistency between a first data acquisition and a subsequent, i.e., second, data acquisition, taken at a later stage in the monitoring of the progression of the disease or therapy. It will be appreciated that the second data acquisition may be days, weeks, months, or even years after the first data acquisition. That is, the second data acquisition may be characterised as occurring a significant length of time after the first data acquisition.

In one example, it may be desirable to maintain a consistent position, and/or orientation, etc. of the relevant radiography apparatus components. In another example, it may be desirable to maintain a consistent patient pose. In yet another examples, it may be desirable to achieve consistent collimation. It will be appreciated that this list is non-exhaustive and there may be other aspects of the first and second data acquisitions which may be desired to keep consistent. It will also be appreciated that aspects which are desirable to maintain as consistent, whether listed above or not, may be kept consistent in isolation or in combination with other aspects.

In the first data acquisition, the radiographic imager 101 captures a first radiographic image of the target. The first radiographic image comprises a landmark feature. In some embodiments, in the first data acquisition, the second image sensor 114 captures a first positional image of the target. Suitably, the first positional image corresponds to the first radiographic image.

Prior to the second data acquisition, when a radiographer may be attempting to monitor the progression of the disease or therapy, the controller 122 controls the display 128 to display a user interface for aiding the radiographer in maintaining consistency between the first data acquisition and the second data acquisition, taken at a later stage in the monitoring of the progression of the disease or therapy, i.e., days, weeks, months or years after the first data acquisition. In effect, by using the displayed user interface, the radiographer can check and improve the consistency of the aspects which are desirable to maintain as consistent, e.g., the position and/or orientation of the relevant radiography apparatus components, the patient pose, the collimation, etc. The user interface may be displayed in response to receiving a user input from the radiographer, or may be displayed automatically when it the controller 122 determines that there is a corresponding first data acquisition, i.e., when the controller 122 determines that the present data acquisition is a second data acquisition related to the first data acquisition.

The user interface displays the landmark feature at a location corresponding to an internal location of the landmark feature. This provides the radiographer with a display for checking the quality of the second data acquisition. For example, the radiographer and/or the radiography apparatus can check that the landmark feature, i.e., the feature of interest, will be consistently, clearly and completely captured in the second data acquisition. This also provides the radiographer with a display for checking the consistency between the first data acquisition and the second data acquisition taken at a later stage in the monitoring of the progression of the disease or therapy (i.e., days, weeks, months or years after the first data acquisition). This also provides improved consistency and quality of the second data acquisition. Improved quality and consistency can be achieved by manually or automatically adjusting, e.g., the position and/or orientation of the relevant radiography apparatus components, the patient pose, the collimation, etc.

It will be appreciated that, to improve monitoring of the progression of a disease or therapy, it is desirable to maintain consistency between a first data acquisition and a subsequent, i.e., second, data acquisition, taken at a later stage in the monitoring of the progression of the disease or therapy. For example, it is desirable to maintain a consistent position and orientation of the relevant radiography apparatus components, a consistent patient pose, and a consistent collimation. Furthermore, in some instances, the size of the landmark feature may require a larger collimation than usual. It will be appreciated that the second data acquisition may be weeks, months, or even years after the first data acquisition. That is, the second data acquisition may be characterised as occurring a significant length of time after the first data acquisition.

In the first data acquisition, the radiographic imager 101 captures a first radiographic image of the target. The first radiographic image comprises a landmark feature. In some embodiments, in the first data acquisition, the second image sensor 114 captures a first positional image of the target. Suitably, the first positional image corresponds to the first radiographic image.

Prior to the second data acquisition, when a radiographer may be attempting to monitor the progression of the disease or therapy, the controller 122 controls the display 128 to display a user interface for aiding the radiographer. The user interface may be displayed in response to receiving a user input from the radiographer, or may be displayed automatically when it the controller 122 determines that there is a corresponding first data acquisition, i.e., when the controller 122 determines that the present data acquisition is a second data acquisition related to the first data acquisition.

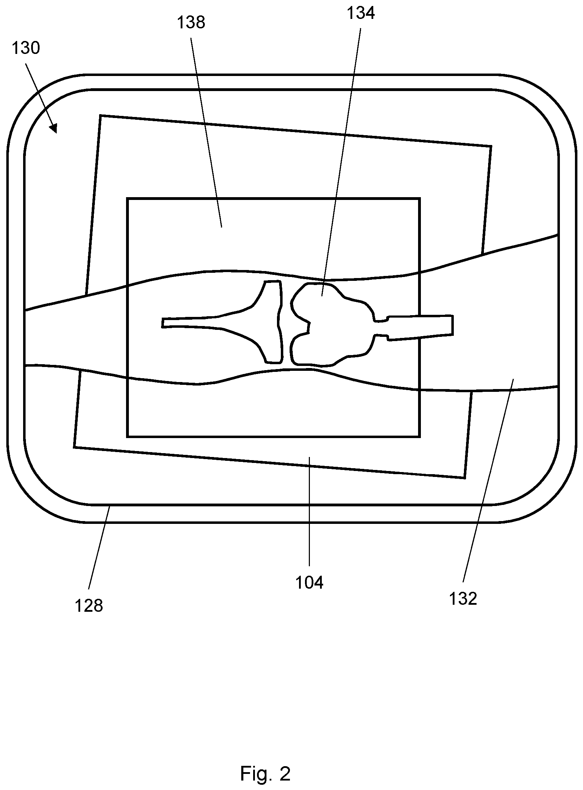

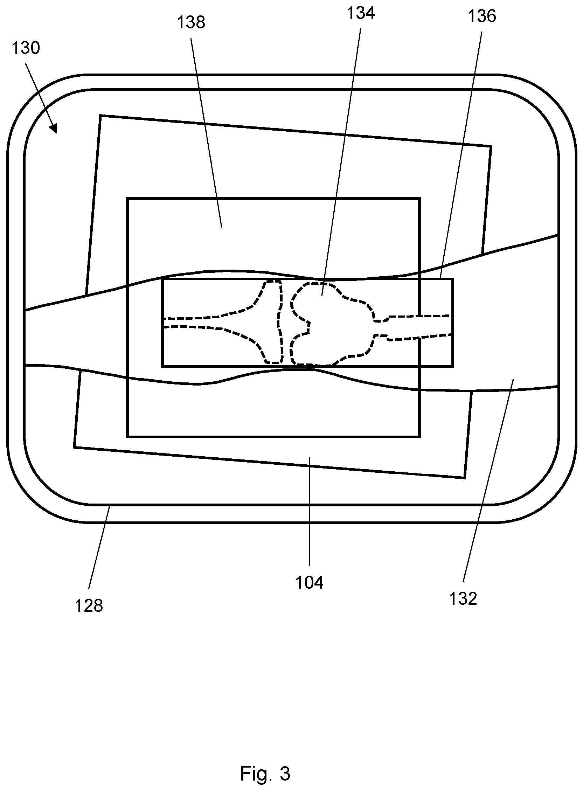

Figs. 2 to 5 show examples of possible user interfaces. In Figs. 2 and 3, the controller 122 controls the display 128 to display a user interface (UI) 130 comprising displaying a live image (or current scene) 132 of the target currently being captured by the second image sensor 114. The UI 130 also displays a landmark feature 134, 136. The landmark feature 134, 136 is displayed overlaid on the live image 132, at a location based on the determined spatial correspondence of the first image sensor 104 with respect to the second image sensor 114.

In Fig. 2, the landmark feature is displayed using a generated, e.g., graphical, representation 134 of the landmark feature. In Fig. 3, the landmark feature is displayed using a bounding box 136 corresponding to the landmark feature. As shown in Fig. 3, optionally, a generated representation 134 of the landmark feature can also be displayed.

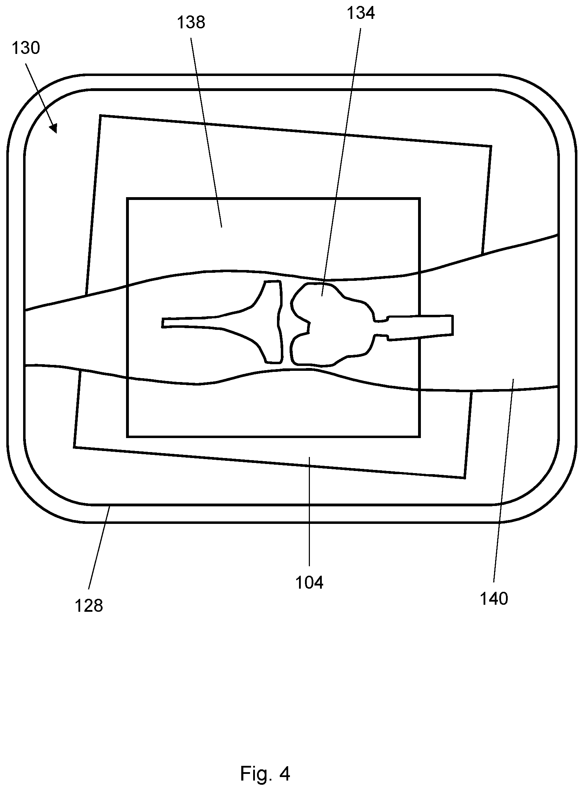

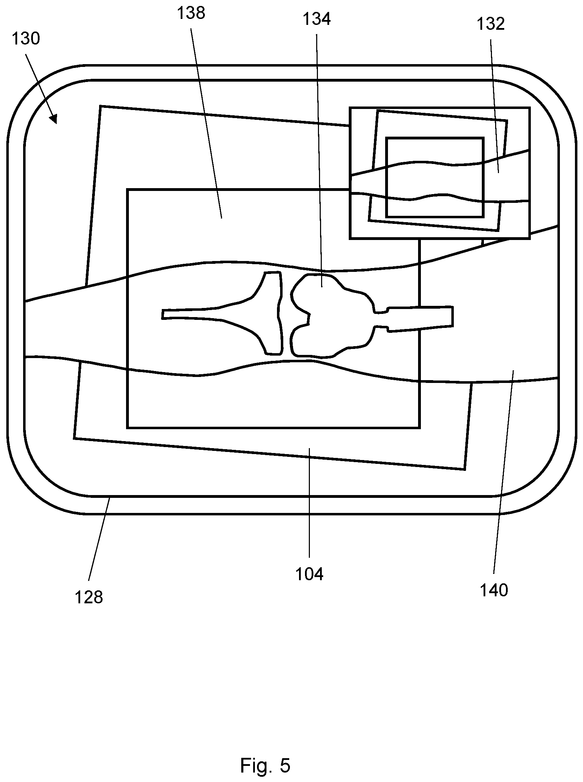

In Figs. 4 and 5, the controller 122 controls the display 128 to display a user interface (UI) 130 comprising displaying the first positional image 140, which was captured in the first data acquisition and which corresponds to the first radiographic image, overlaid with the identified landmark feature at a location based on the determined spatial correspondence. In such examples, the first positional image is obtained from the memory 124. Similarly to Fig. 3, the landmark feature can also be displayed on the first positional image 140 using a bounding box 136 and/or a generated representation 134 of the landmark feature.

In Figs. 4 and 5, the controller 122 controls the display 128 to display a user interface (UI) 130 comprising displaying the first positional image 140 overlaid with the identified landmark feature at a location based on the determined spatial correspondence. Similarly to Fig. 3, the landmark feature can also be displayed on the first positional image 140 using a bounding box 136 and/or a generated representation 134 of the landmark feature.

In Fig. 5, the first positional image 140 is displayed overlaid with the identified landmark feature. Additionally, the current scene 132 is shown in a sub-window in a portion of the UI 130. Suitably, the radiographer is provided with means to compare the patient's pose corresponding to the previous (i.e., first) image acquisition to the current pose, since the UI 130 can display the corresponding previous positional image 140 on the display 128 while showing the current scene 132. In another example, when the current scene 132 overlaid with the identified landmark feature is displayed, the first positional image 140 can be shown in a sub-window in a portion of the UI 130.

It will be appreciated that in order to overlay the landmark feature, the landmark feature must be identified from the first radiographic image. Identifying the landmark features may utilise a number of approaches. In some examples the landmark feature may be identified using classical image analysis techniques that would be familiar to those in the art. In a preferred approach, however, it is envisaged that the controller 122 may run a computer vision or machine learning algorithm to identify the landmark feature in the first radiographic image. It will be appreciated that a machine learning approach to landmark feature localization can be realized by training of a suitably designed neural network to e.g., localize / segment the respective structures in radiographic images. In some examples, identifying the landmark feature in the first radiographic image may include edge detection.

In some examples, the landmark feature is an implant; for example, a medical implant; more specifically, e.g., an orthopedic implant. Suitably, the landmark feature is any internal structure that can be assessed to monitor the progression of a disease or therapy. In some examples, one landmark feature can be identified from the first radiographic image. In other examples, multiple landmark features, of the same or different types, can be identified from the first radiographic image.

In some examples, the controller 122 can generate a graphical representation of the landmark feature, e.g., by generating an outline of the landmark feature using detected edges. In some examples, the controller 122 can generate a bounding box that contains the landmark feature.

In examples in which the apparatus 100 comprises the light 115, an illuminated area 138 can also be shown in the first positional image 140 and/or the live image 132 for assisting collimation. The illuminated area 138 is expected to substantially correspond to the target area 106. The illuminated area 138 can be detected using computer vision or machine learning algorithms, utilising a similar approach to that used to identify the landmark feature, as described herein.

Furthermore, it will be appreciated that in order to overlay the landmark feature, a spatial correlation must be established between the first image sensor 104 and second image sensor 114. In other words, the controller 122 is configured to determine a spatial correlation of the first image sensor 104 with respect to at least one of the live scene being captured 132 and the previously captured first positional image 140. A number of approaches may be employed. Advantageously, to ensure the landmark feature is overlaid accurately, the anatomy of the target 108 is analysed in the first radiographic image 148 and the anatomy of the target 108 is analysed in the first positional image 140 or the live image 132. This anatomy analysis is used to determine a spatial correspondence of the first image sensor 104 with respect to the second image sensor 114, as described herein.

In one example, the first image sensor 104 may be localized due to known geometrical constellations, e.g. when placing it below the table in a tray of known location and/or orientation relative to the second image sensor 114. In this case, the first image sensor 104 does not need to be visible, but the second image sensor 114 needs to be either calibrated or located in a known geometrical constellation to the first image sensor 104.

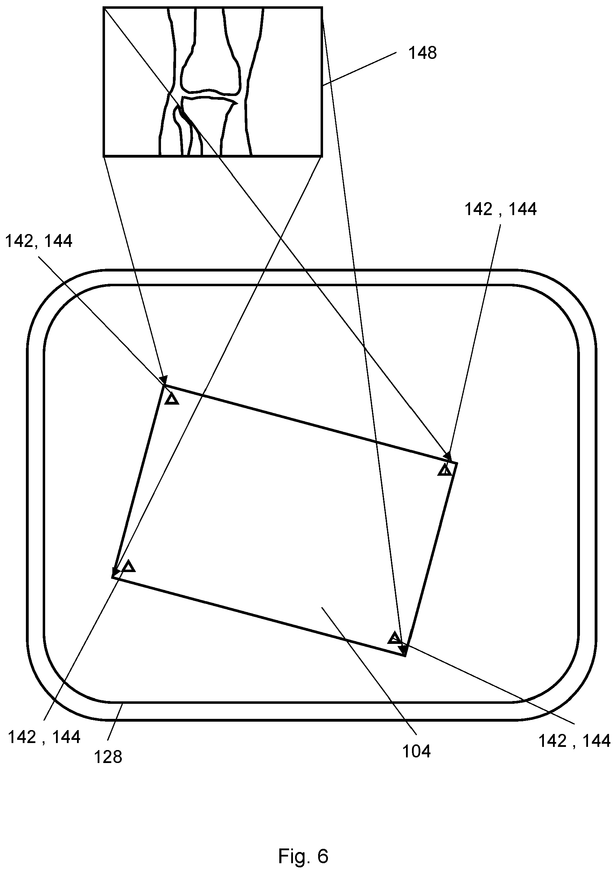

In another example, suitable for when the first image sensor 114 will be visible to the second image sensor 114 - i.e., an image captured by the second image sensor 114 comprises an at least partial image of the first image sensor 104 - then the first image sensor 104 may comprise one or more markers 142, as shown in Fig. 6. The markers may be particular shapes, colours, QR codes, and so on, and may be positioned on the first image sensor 104 in a known geometric arrangement (e.g., on the corners). Suitably, the controller 122 may perform image analysis to identify the position of the markers in the image. The analysis may be performed on both the live image 132 being captured by the second image sensor 114, or the first positional image 140 previously captured.

In another example, determining the spatial correspondence of the first image sensor 104 comprises analysing an anatomy of the target 108. That is, the controller 122 may perform image analysis on the first radiographic image 148 to determine an orientation of the anatomy with respect to the first image sensor 104. In some examples, the controller 122 also performs image analysis on the first positional image 140 or the live image 132 to determine an orientation of the same anatomy with respect to the first image sensor 104. For example, if the first radiographic image 148 shows a tibia and femur either side of a knee joint, the controller 122 may determine an orientation of an upper and/or lower leg corresponding to the tibia and femur, and then determine an orientation and position of the upper and lower leg in the first positional image 140 that was taken at the same time as the first radiographic image 148 or in the live image 132. The controller 122 may determine the spatial position of the first image sensor 104 within the first positional image 140 or within the live image 132. The controller 122 can overlay the landmark feature 134, 136 onto the first positional image 140 or the live image 132 using the determined spatial relationship.

Analysing the anatomy of the target may utilise a number of approaches but it is particularly envisaged that the controller 122 may run a computer vision, or machine learning, algorithm to detect the anatomy and thereby localise the first image sensor 104 with respect to the first positional image 140. It will be appreciated that a machine learning approach to anatomy localization can be realized by training of a suitably designed neural networks to e.g. localize / segment the respective structures in the images.

In yet another example, the first image sensor 104 comprises a set of radiofrequency beacons 144, as shown in Fig. 6. The beacons 144 may emit at radiofrequency, for example a Bluetooth^{™} frequency or an ultra-wide band frequency. Different beacons 144 may be arranged to emit at slightly different frequencies, and/or in a well-defined temporal pattern. It will be appreciated that the beacons 144 are arranged in a predefined spatial pattern, such as the corners, like the markers 142. In this example, the apparatus 100 comprises one or more radiofrequency receivers coupled to the controller 122. Suitably, determining the spatial correspondence of the first image sensor 104 within the first positional image 140, comprises analysing the signals received from the radio frequency beacons 144 to determine the spatial arrangement from the temporal pattern. For example, by triangulation. Advantageously, in this arrangement the first image sensor 104 does not need to be visible.

In yet another example, a spatial relationship between the radiated area on the first image sensor 104 and the corresponding image captured by the second image sensor 114 is established by retrieving a security identifier, collimation parameters, and a plane of the first image sensor 104 relative to radiation generator 102 (e.g. taking the tube-angulation into account) from the system, calculating corner points of the radiated region on the first image sensor 104 in the first positional image 140 (using known spatial correspondence to the radiation generator 102), manually / automatically localizing the collimated area in the first radiographic image 148 and deriving the corresponding corner-points from this information, and resolving the potential ambiguity regarding discrete rotations when finding the correspondence between the corner points in the radiographic image and the positional image by automatically detecting a reference direction in both images (e.g. direction towards the patient's head) using computer vision or machine learning.

Having localized the first image sensor 104 relative to the first positional image 140 or the live captured image 132, a geometrical relationship between the first radiographic image 148 and an image captured by the second image sensor 114 can be established by determining the affine transformation which maps the corners of the detector pixel-matrix to the corresponding points in the camera image. This is shown in Fig. 6. The radiographic image 148 pixel-matrix can be transformed onto the image from the second image sensor 114 using this affine transformation. The transformed radiographic image 148 may be restricted to the collimated area using the known / detected shutter configuration in the X-ray pixel-matrix. Finally, the landmark feature 134, 136 can be overlaid onto the second image sensor image 132, 140 using the determined spatial relationship.

Furthermore, the visualization may be refined by using a perspective transformation instead of an affine one if, e.g., using also depth information (via an RGB-D camera) or system parameters (e.g. source to image distance, tube angulation, etc.).

In an example where a depth of the target 108 anatomy may be determined (using e.g., a depth camera), the depth surfaces of the anatomy corresponding to the current patient pose and the pose of the first data acquisition can be registered. The deviation between the poses (e.g. 8° difference of leg-rotation, 10° difference in flexion) can be derived from the registration transformation and anatomical knowledge (e.g. by detecting landmarks on the anatomy by a correspondingly trained neural network).

In an example, such measurements of a pose of the target 108, based on the target as captured in the first radiographic image 148, may be displayed on the user interface. Put another way, the UI 130 may comprise information on the pose of the target 108.

Suitably, the radiographer may be presented with quantitative information on pose as part of the UI to supplement any qualitative visualisation. That is, the radiographer may be presented with results from automatic assessment of positioning consistency from which to directly infer which positioning degrees of freedom need to be adapted, in which direction and how much, etc. Put another way, the controller 122 may determine a pose correction based on the determined pose in the first radiographic image 148, and control to display information corresponding to the determined pose correction (as part of the first radiographic image 148).

The radiographer can also use the information displayed on the UI 130 adjust the collimation. For example, as shown in Figs. 2 to 5, part of the landmark feature 134, 136 is outside of the illuminated area 138. Therefore, the radiographer may adjust the patient pose or the collimation to ensure the landmark feature 134, 136 is within the illuminated area 138. Thus, the radiographer can use the information displayed on the UI 130 to instruct adjustment of the target 108 and/or apparatus 100 in order to allow for correct imaging of the target 108. In some examples, the adjustment of the apparatus 100 can be carried out automatically. In some examples, the controller 122 is configured to adjust a collimation parameter, or other apparatus parameter, based on the displayed UI 130 to ensure the landmark feature 134, 136 is within the illuminated area 138.

In summary, there is described herein a radiography apparatus 100 comprising a controller 122 configured to identify a landmark feature from a first radiographic image 148; and to determine a spatial correspondence of a first radiographic image sensor 104 with respect to a second positional image sensor 114. The controller 122 is further configured to, prior to a second data acquisition, control a display 128 to display a user interface 130 comprising displaying at least one of: a live image 132 of a target 108 currently captured by the second image sensor 114 overlaid with the identified landmark feature 134, 136 at a location based on the determined spatial correspondence; and a first positional image 140 overlaid with the identified landmark feature 134, 136 at a location based on the determined spatial correspondence.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. Any reference signs in the claims should not be construed as limiting the scope.

## Claims

1. A radiography apparatus (100), comprising:
a radiographic imager (101) configured to capture a radiographic image of a target (108), the radiographic imager comprising a radiation generator (102) and a first image sensor (104);
a positional imager comprising a second image sensor (114) configured to capture a positional image of the target;
a display (128);
a memory (124); and
a controller (122) configured to:
obtain a first radiographic image (148) of the target from the memory, wherein the first radiographic image was captured in a first data acquisition, and the first radiographic image comprises a landmark feature;
identify the landmark feature in the first radiographic image;
determine a spatial correspondence of the first image sensor with respect to the second image sensor; and
prior to a second data acquisition, control the display to display a user interface (130) comprising displaying a live image (132) of the target currently captured by the second image sensor overlaid with the identified landmark feature (134, 136) at a location based on the determined spatial correspondence.

2. The apparatus of claim 1, wherein the positional imager comprises a depth sensor.

3. The apparatus of any preceding claim, wherein the landmark feature is identified by inputting the first radiographic image into a trained machine learning model.

4. The apparatus of any preceding claim, wherein the identified landmark feature is an implant.

5. The apparatus of any preceding claim, wherein the first image sensor comprises one or more markers (142), and wherein determining the spatial correspondence of the first image sensor comprises determining the location of the markers within the live image.

6. The apparatus of any preceding claim, wherein the determining the spatial correspondence of the first image sensor comprises analysing an anatomy of the target in the live image and comparing to an anatomy of the target in the first radiographic image.

7. The apparatus of any preceding claim wherein the controller is further configured to:
obtain a first positional image corresponding to the first radiographic image from the memory, wherein the first positional image was captured in the first data acquisition; and
control the display to display the first positional image.

8. The apparatus of any preceding claim wherein the displaying the live image overlaid with the identified landmark feature comprises displaying a bounding box (136) corresponding to the landmark feature.

9. The apparatus of any preceding claim wherein the displaying the live image overlaid with the identified landmark feature comprises displaying a generated representation (134) of the landmark feature.

10. The apparatus of any preceding claim, wherein the radiographic imager further comprises a collimator, and wherein the controller is further configured to adjust a collimation parameter.

11. The apparatus of any preceding claim, wherein the controller is further configured to:
determine a pose of the target captured in the first radiographic image;
determine a pose of the target captured in the live image;
determine a pose correction based on the determined pose in the first radiographic image and the determined pose in the live image, and
wherein the user interface comprises displaying information corresponding to the determined pose correction.

12. A radiography apparatus (100), comprising:
a radiographic imager (101) configured to capture a radiographic image of a target (108), the radiographic imager comprising a radiation generator (102) and a first image sensor (104);
a positional imager comprising a second image sensor (114) configured to capture a positional image of the target;
a display (128);
a memory (124); and
a controller (122) configured to:
obtain a first radiographic image (148) of the target from the memory, wherein the first radiographic image was captured in a first data acquisition, and the first radiographic image comprises a landmark feature;
obtain a first positional image (140) corresponding to the first radiographic image from the memory, wherein the first positional image was captured in the first data acquisition;
identify the landmark feature in the first radiographic image;
determine a spatial correspondence of the first image sensor with respect to the second image sensor; and
prior to a second data acquisition, control the display to display a user interface (130) comprising displaying the first positional image overlaid with the identified landmark feature (134, 136) at a location based on the determined spatial correspondence.

13. The apparatus of claim 12 wherein the controller is further configured to:
control the display to display a live image (132) of the target currently captured by the second image sensor.

14. A method for controlling a radiography apparatus, comprising:
obtaining a first radiographic image of a target from a memory, wherein the first radiographic image was captured by a first image sensor in a first data acquisition, and the first radiographic image comprises a landmark feature;
identifying the landmark feature in the first radiographic image;
determining a spatial correspondence of the first image sensor with respect to a second image sensor configured to capture a positional image of the target; and
prior to a second data acquisition, controlling the display to display a user interface comprising displaying a live image of the target currently captured by the second image sensor and overlaid with the identified landmark feature at a location based on the determined spatial correspondence.

15. The method of claim 14, further comprising:
adjusting a collimation parameter of a collimator based on the displayed user interface.
